# EUROPÄISCHE PATENTSCHRIFT

(11) **EP 4 129 376 B1**
(45) Veröffentlichungstag und Bekanntmachung des Hinweises auf die Patenterteilung: **24.09.2025**
(21) Anmeldenummer: 22185647.9
(22) Anmeldetag: 19.07.2022
(51) Int. Cl.: A61M 16/00

(54) **VORRICHTUNG ZUR BEATMUNG EINES PATIENTEN MIT GEREGELTEM DRUCK-ÜBERGANG**
DEVICE FOR VENTILATING A PATIENT WITH CONTROLLED PRESSURE TRANSITION
DISPOSITIF DE VENTILATION D'UN PATIENT À TRANSITION DE PRESSION RÉGULÉE

(30) Priorität: 05.08.2021 DE 102021120415
(43) Veröffentlichungstag der Anmeldung: 08.02.2023
(73) Patentinhaber: Löwenstein Medical Technology S.A., 2557 Luxembourg (LU)
(72) Erfinder: Schwaibold, Matthias, 76228 Karlsruhe (DE)
(74) Vertreter: Löwenstein Medical IP

(56) Entgegenhaltungen:
- EP-B1- 2 514 469
- WO-A2-2015/027980
- DE-A1- 102010 045 839
- DE-B3- 102005 063 665

## Beschreibung

Die Erfindung bezieht sich auf Vorrichtungen zur Beatmung die zumindest einen einstellbaren positiven exspiratorischen Druck ("expiratory positive airway pressure", EPAP), als auch einen einstellbaren inspiratorischen positiven Atemwegsdruck ("inspiratory positive airway pressure", IPAP) vorgeben. Dabei werden die Atemzüge beispielsweise vom Patienten getriggert. Der EPAP kann dabei einem PEEP entsprechen. PEEP bedeutet "Positive End-Expiratory Pressure" (positiver endexspiratorischer Druck) und bezeichnet den positiven Druck, der am Ende des Ausatmens in der Lunge verbleibt. Die Erfindung bezieht sich auf eine Vorrichtung, welche den IPAP gezielt auf den EPAP absenkt und so die Zeitdauer und den Verlauf der Ausatemphase steuert.

Derartige Vorrichtungen können insbesondere im Zusammenhang mit Beatmungsgeräten zum Einsatz kommen, die für Patienten mit COPD-Erkrankungen verwendet werden. Typische Krankheitsbilder sind Emphysem und chronisch obstruktive Bronchitis. Diese Erkrankungen führen dazu, dass es im Rahmen einer Beatmung in der Exspiration zu einem Kollaps kleiner Atemwege kommen kann. Entsprechend ist die Exspiration unvollständig und ein erhöhter intrapulmonaler Druck (auch intrinsischer PEEP) besteht. Die Behinderung der Ausatmung führt zu einem intrathorakalen Druckanstieg. Daraus folgt eine Minderbelüftung der Lunge. Die US 2006/011195 A1 offenbart, dass zunächst ein Basisdruck vorgegeben wird, welcher dann auf ein Ausatemniveau abgesenkt und anschließend auf einen Spitzenwert angehoben wird. Daraufhin wird der Druck wieder auf den Basisdruck abgesenkt. Die US 2006/011195 A1 offenbart drei unterschiedliche Druckniveaus für die Beatmung. Die Druckabsenkungen erfolgen hier passiv.

Die WO 99/45989 offenbart eine Ausatemerleichterung, bei der der exspiratorische Basisdruck (EPAP) noch unterschritten wird. Hierbei wird abhängig von dem Atemgasfluss ein Verstärkungsfaktor ermittelt und die Ausatemerleichterung wird mit dem Verstärkungsfaktor multipliziert.

Die US 3,961,627 offenbart die Automatisierung einer druckkontrollierten oder volumenkontrollierten Beatmung. Hierbei werden vier Phasen unterschieden, wobei die Phasen III und IV der Exspiration dienen, wobei die die Zeitdauer der Phase IV nie kürzer ist als die Dauer der Phase III ist, um eine Überblähung der Lunge (air trapping) zu vermeiden.

Die EP 2 542 286 B1 offenbart einen kontrollierten Druckverlauf vom IPAP-Druck auf den EPAP-Druck, bei dem der Druck in der Ausatmungsphase sogar zeitweise erhöht wird.

Die EP 2 514 469 B1 offenbart einen kontrollierten Druckabfall vom IPAP-Druck auf den PEEP- oder EPAP-Druck in drei Phasen. In einer ersten Phase ist der Druckabfall schneller als in einer zweiten Phase. Nach Beendigung der zweiten Phase erfolgt der Druckabfall in einer dritten Phase schneller als in der zweiten Phase. Der PEEP-Druck wird erst zum Ende der Ausatemzeit erreicht.

Die DE 10 2005 063 665 B3 offenbart eine Vorrichtung zur Drucksteuerung bei Beatmungsgeräten mit einer Steuereinheit, die die zeitliche Lage von Inspirations- und Exspirationsphasen auswerten und eine Druckabsenkung innerhalb der Inspirationsphase vor dem Beginn der Exspirationsphase vorgeben kann. Die Druckabsenkung wird nach einem Überschreiten eines Flussmaximums durchgeführt, wobei der Druck nach der Durchführung der Druckabsenkung für eine vorgebbare oder automatisch ermittelte Spanne auf einem im Wesentlichen gleichbleibenden unteren Druckniveau gehalten wird und vor dem Ende der Exspirationsphase ein Druckanstieg durchgeführt wird.

Aufgabe der vorliegenden Erfindung ist es, eine Vorrichtung anzugeben, die durch eine intelligente und störungstolerante Gerätesteuerung eine ausreichende Exspiration unterstützt, idealerweise dergestalt, dass die kollapsiblen Areale der Lunge zumindest während eines ersten Teils der Rampenzeit geöffnet bleiben und dadurch teilentlüftet werden und noch ausreichend Zeit bleibt, um die nicht kollabierten Areale der Lunge auf dem EPAP-Niveau zu entlüften.

Diese Aufgabe wird durch den Gegenstand des unabhängigen Anspruchs gelöst.

Die Erfindung betrifft eine Vorrichtung gemäß dem unabhängigen Anspruch, nämlich eine Vorrichtung zur Beatmung, die eine steuerbare Atemgasquelle und eine programmierbare Steuereinheit aufweist, wobei die programmierbare Steuereinheit zum Durchführen der folgenden Schritte eingerichtet ist: Bestimmen eines Atemgasflusses, wobei aus dem Atemgasfluss bestimmt wird, ob eine Inspiration oder eine Exspiration vorliegt; Regeln eines Drucks für die Inspiration (IPAP) und die Exspiration (EPAP). Dabei berücksichtigt die Steuereinheit eine typische Exspirationszeit, wobei die Steuereinheit die typische Exspirationszeit über n Atemzüge ermittelt und die so ermittelte Exspirationszeit berücksichtigt, wobei die Steuereinheit den Druck vom IPAP auf den EPAP unter Berücksichtigung der typischen Exspirationszeit derart absenkt, dass der Druckabfall auf den EPAP zumindest zu 85 % bereits nach einem Anteil der typischen Exspirationszeit erreicht wird, der im Bereich von 40 % bis 60 % der typischen Exspirationszeit liegt, wobei der EPAP nach Beendigung des Druckabfalls bis zum Ende der typischen Exspirationszeit vorgegeben wird.

Gemäß einer nicht beanspruchten Alternative kann die Steuereinheit anstelle der (über n Atemzüge ermittelten) typischen Exspirationszeit auch eine fest vorgegebene Exspirationszeit, die entweder explizit oder implizit über die Vorgabe einer Inspirationszeit und einer Atemfrequenz vorgegeben ist, berücksichtigen.

Es ist bevorzugt und vorteilhaft, dass der Druck vom IPAP auf den EPAP in Form einer adaptiven Druckrampe abgesenkt wird, wobei der Druck den EPAP nach 40 % bis 60 %, bevorzugt nach 50 % der typischen Exspirationszeit erreicht.

Insbesondere ist die Vorrichtung dazu geeignet und ausgebildet, den Druckabfall auf den EPAP zumindest zu 90 % bereits nach einem Anteil oder Bruchteil der typischen Exspirationszeit zu erreichen.

Auch ist die Vorrichtung dazu geeignet und ausgebildet, eine adaptive Druckrampe vorzugeben, bei welcher der Druck den EPAP nach 40 % bis 60 %, bevorzugt nach 50 % der typischen Exspirationszeit erreicht.

Der Abfall auf den EPAP kann zu zumindest 80 %, bevorzugt zu 90 % und besonders bevorzugt zu 100 %, nach 40 % bis 60 %, bevorzugt nach 50 % der typischen Exspirationszeit erreicht sein.

Möglich ist auch, dass der EPAP nach einem Anteil der typischen Exspirationszeit erreicht wird, der bei 50 % liegt.

Die Vorrichtung weist zumindest beispielhaft einen Flusssensor und/oder einen Drucksensor oder äquivalente Bestandteile auf.

In einer vorteilhaften Ausgestaltung ist die Vorrichtung so ausgestaltet und eingerichtet, dass nach Erkennung einer Ausatemphase der Druck vom IPAP auf den EPAP so verringert wird, dass der Druck als dynamisch geregelter Gegendruck gegen den Atemgasfluss der Exspiration angelegt wird, wodurch die unteren Atemwege bei COPD-Patienten mit exspiratorischer Flusslimitation für etwa die Hälfte der typischen Exspirationszeit geschient bleiben und nicht kollabieren.

In einer vorteilhaften Weiterbildung ist die Vorrichtung dazu geeignet und ausgebildet, den Druck vom IPAP auf den EPAP als einen nichtlinearen Abfall mit asymptotischer Näherung an den EPAP zu verringern.

In einer anderen Weiterbildung ist die Vorrichtung dazu geeignet und ausgebildet, den Druck vom IPAP auf den EPAP so zu verringern, dass 90 % des Druckabfalls nach etwa der Hälfte der Exspirationszeit erreicht ist.

In einer vorteilhaften Ausgestaltung ist die Vorrichtung dazu geeignet und ausgebildet, den Druck nur zu Beginn der Exspiration zu regeln und nach Erreichen des EPAP auf diesem Niveau zu belassen.

Weiterhin ist die Vorrichtung so eingerichtet und ausgebildet, dass die Steuereinheit eine Erhöhung des exspiratorischen Widerstands erkennen kann, beispielsweise über eine vorzeitige Abflachung der exspiratorischen Flusskurve oder eine Widerstandsmessung mittels Forced-Oscillation-Technologie. Dabei erfolgt der Druckabfall vom IPAP auf den EPAP unter Berücksichtigung des exspiratorischen Widerstands, beispielsweise indem die Rampe des Druckabfalls flacher eingestellt wird.

Die Vorrichtung ist weiterhin beispielsweise so eingerichtet und ausgebildet, dass die Steuereinheit einen erhöhten Restfluss zum Ende der Exspiration erkennt, beispielsweise indem die aktuellen Flusswerte mit den Flusswerten vorangegangener Exspirationen verglichen werden, und der Druckabfall vom IPAP auf den EPAP unter Berücksichtigung des erhöhten Restflusses zum Ende der Exspiration erfolgt, beispielsweise indem die Rampe des Druckabfalls steiler eingestellt wird.

Alternativ oder ergänzend ist die Vorrichtung so eingerichtet und ausgebildet, dass die Steuereinheit eine Variation der Steilheit des Druckabfalls, beispielsweise von Atemzug zu Atemzug, durchführt und ermittelt, bei welcher Steilheit des Druckabfalls die beste Entlüftung der Lunge erfolgt. Dies ermittelt die Steuereinheit beispielsweise über eine Berechnung des exspiratorischen Tidalvolumens oder eine Messung des verbleibenden Restflusses am Ende der Exspiration. Dieser Lernvorgang kann während der Beatmung regelmäßig wiederholt werden.

Vorzugsweise ist die Vorrichtung so eingerichtet und ausgebildet, dass die Steuereinheit eine Änderung der Steilheit des Druckabfalls, beispielsweise nach wie viel Prozent der Exspirationszeit der EPAP erreicht sein soll, entsprechend einer Vorgabe durchführt, die über eine Datenschnittstelle eingegeben wird. Die Vorgabe kann dabei von einem Anwender oder automatisch im Rahmen einer Fernsteuerung des Geräts über ein Netzwerk oder die Cloud erfolgen. Die Vorgabe kann dabei auch von einem Anwender oder automatisch im Rahmen einer Steuerung oder Eingabe über eine Bedieneinheit, basierend auf Atemflusskurven oder weiteren Sensorsignalen oder anhand des Feedbacks des Patienten, erfolgen. Die Vorgabe kann manuell erfolgen oder automatisch durchgeführt werden. Die Vorgabe kann mindestens einen der folgenden Parameter berücksichtigen: Alter, Größe, Erkrankung, Therapieziel, Lungenvolumen, Lungencompliance, Lungenresistance, Atemanstrengung, Atemnot.

Insbesondere ist Vorrichtung so eingerichtet und ausgebildet, dass die Steuereinheit eine Änderung der Steilheit des Druckabfalls basierend auf sensorischen Signalen - beispielsweise von Effort-Gurten, Zwerchfell-EMG, Ösophagusdruck-Sonden, Bodyplethysmografie oder Elektroimpedanztomografie - steuert. Die sensorischen Signale geben dabei ein Maß für die Lungenfüllung am Ende der Inspiration und/oder Exspiration und/oder ein Maß für die Veränderung der Lungenfüllung im Verlauf der Inspiration und/oder Exspiration wieder. Dieses Maß wird von der Steuereinheit zur Veränderung der Steilheit des Druckabfalls genutzt. Insbesondere ändert die Steuereinheit die Steilheit des Druckabfalls auch anhand der zeitlichen Veränderung des Maßes oder der Übereinstimmung dieser zeitlichen Veränderung mit der zeitlichen Veränderung der Steilheit des Druckabfalls.

Die Vorrichtung kann auch so eingerichtet und ausgebildet sein, dass die Steuereinheit eine Änderung der Steilheit des Druckabfalls durchführt, wenn die Druckunterstützung angepasst wird, also die Differenz zwischen IPAP und EPAP geändert wird. Die

Druckunterstützung kann entweder manuell oder automatisch, beispielsweise bei antizyklischer Servoventilation oder bei Zielvolumensteuerung, angepasst werden. Bei einer größer werdenden Druckunterstützung wird die Steilheit des Druckabfalls typischerweise reduziert, sodass der EPAP erst zu einem späteren Zeitpunkt in der Exspiration erreicht wird. Damit wird ein Teil des stärker abfallenden Therapiedrucks kompensiert und die Atemwege werden gegen einen Kollaps stärker geschient.

Die Vorrichtung kann auch so eingerichtet und ausgebildet sein, dass die Steuereinheit eine Änderung der Steilheit des Druckabfalls durchführt, wenn der EPAP angepasst wird. Bevorzugt wird die Steilheit erhöht, wenn der EPAP erhöht wird, da durch den höheren EPAP ohnehin eine stärkere Schienung der unteren Atemwege erreicht wird.

Vorzugsweise ist die Vorrichtung auch so eingerichtet und ausgebildet, dass die Steuereinheit eine typische Inspirationszeit über n Atemzüge ermittelt und den Druck vom EPAP auf den IPAP unter Berücksichtigung der charakteristischen Inspirationszeit derart anhebt, dass der IPAP nach einem Anteil der typischen Inspirationszeit erreicht wird, der im Bereich von 20 % bis 40 % liegt.

Die Vorrichtung ist weiterhin beispielsweise so eingerichtet und ausgebildet, dass die Steuereinheit den Druck vom EPAP auf den IPAP unter Berücksichtigung der typischen Inspirationszeit derart anhebt, dass der IPAP nach einem Anteil der typischen Inspirationszeit erreicht wird, der im Bereich von 30 % liegt.

Es ist möglich und bevorzugt, dass die Steuereinheit den Druck vom EPAP auf den IPAP derart anhebt, dass ein nichtlinearer Anstieg mit asymptotischer Näherung an den IPAP resultiert. Es ist auch möglich und bevorzugt, dass die Steuereinheit den Druck vom EPAP auf den IPAP derart anhebt, dass etwa 90 % des Anstiegs nach 30 % der Inspirationszeit erreicht ist.

Die Vorrichtung kann dazu geeignet und ausgebildet sein, die Druckveränderung (EPAP zu IPAP oder IPAP zu EPAP) mit fester Dauer oder Geschwindigkeit oder damit assoziierter Stufe vorzugeben.

Vorzugsweise ist die Vorrichtung so eingerichtet und ausgebildet, dass die Steuereinheit die typische Exspirationszeit und/oder die typische Inspirationszeit über zumindest drei Atemzüge oder bevorzugt 10 Atemzüge ermittelt, wobei ein Atemzug eine Inspiration und eine Exspiration umfasst.

Eine solche Anzahl an Atemzügen hat sich als besonders verlässlich für die Identifizierung der typischen Exspirationszeit und/oder der typischen Inspirationszeit erwiesen.

Die Vorrichtung kann ergänzend dazu geeignet und ausgebildet sein, das Atemvolumen der Inspiration (AI) zu bestimmen und die Zeitdauer der Exspiration anzupassen, wenn das Atemvolumen der Exspiration (AE) den Wert des Atemvolumens der Inspiration (AI) erreicht.

Die Erfindung betrifft im Wesentlichen Vorrichtungen zur Beatmung wie APAP-Geräte, Bilevel-Geräte, Servoventilationsgeräte, Geräte für die Heimbeatmung und Intensivbeatmung sowie Notfallbeatmungsgeräte. Die Erfindung kann mit einem Leckageschlauch, einem Einschlauch-Ventilsystem oder einem Doppelschlauchsystem - in Kombination mit den oben genannten Vorrichtungen zur Beatmung - verwendet werden.

Die Erfindung kann bei Vorrichtungen zur Beatmung realisiert sein, die eine spontane und/oder mandatorische Beatmung ermöglichen. Die Beatmungsform der spontanen Beatmung ist als unterstützende Beatmung vorgesehen, bei der der Patient selbst atmet. Er steuert die Atemfrequenz und das Beatmungsgerät unterstützt die Ein- und/oder Ausatmung durch einen voreingestellten Druck. Ausgelöst wird die Beatmung durch einen sogenannten Trigger. Der Patient erzeugt zu Beginn der Einatmung selbst einen Atemgasfluss oder -druck, den das Beatmungsgerät erkennt. Überschreitet der vom Patienten erzeugte Atemgasfluss oder -druck die voreingestellte Schwelle, so schaltet das Gerät auf den Druck für die Ein- und/oder Ausatmung.

Bei der mandatorischen Beatmung gibt das Beatmungsgerät die Abfolge von Ein- und Ausatmung vor. Hier ist die Zeit der Ausatmung also vorbestimmt (und wird nicht als die typische Exspirationszeit ermittelt).

Bei der spontanen Beatmung kann auch - zumindest für einen oder wenige Atemzüge - die Ausatmung durch den Patiententrigger früher beendet werden, sodass möglicherweise der EPAP oder die typische Exspirationszeit noch nicht erreicht ist.

Es wird darauf hingewiesen, dass eine hierin verwendete, zwischen zwei Merkmalen stehende und diese miteinander verknüpfende "und/oder"-Konjunktion stets so auszulegen ist, dass in einer ersten Ausgestaltung lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können. Weitere Vorteile und Merkmale der vorliegenden Erfindung ergeben sich aus der Beschreibung der Ausführungsbeispiele, welche mit Bezug auf die beiliegenden Figuren im Folgenden erläutert werden.

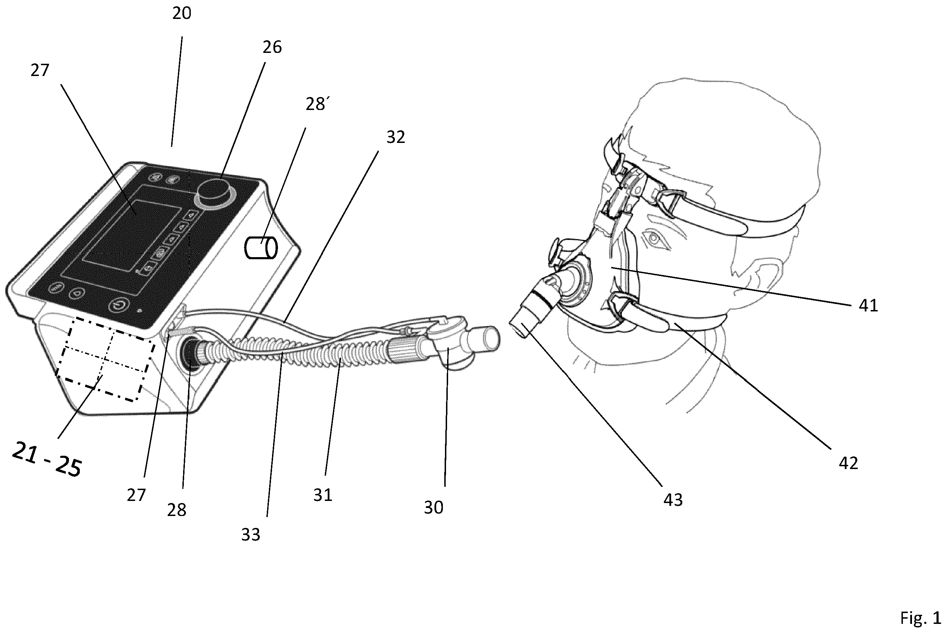

Fig. 1 zeigt eine Vorrichtung 20 mit einer Beatmungsmaske 41 als Patienteninterface. Die Maske 41 ist mit einer Bänderung 42 am Kopf befestigt. Über einen Maskenstutzen 43 kann die Maske 41 mit einem Schlauch verbunden werden.

Die Vorrichtung 20 umfasst eine Atemgasquelle 21, eine Steuereinheit 22, einen Speicher 25, eine Drucksensoreinrichtung 24 und/oder eine Flusssensoreinrichtung 23, einen Atemgasschlauch 31 und ein Patienteninterface, welches hier als die Beatmungsmaske 41 ausgebildet ist. Die Vorrichtung 20 weist zudem eine Bedieneinheit 26 und eine Anzeige 27 auf. Die Vorrichtung 20 weist zumindest einen Stutzen 28 für den Atemgasschlauch 31 auf. Der Stutzen 28 ist für den Anschluss des Atemgasschlauchs 31 in Form eines Einschlauch-Ventilsystems 31 eingerichtet. An den Stutzen 28 kann auch ein Leckageschlauch angeschlossen werden.

Zudem kann an den Stutzen 28 der inspiratorische Zweig eines Doppelschlauchsystems angeschlossen werden. Ein anderer Stutzen 28' dient dem Anschluss des exspiratorischen Zweigs des Doppelschlauchsystems.

Die Vorrichtung 20 kann mit einem Leckageschlauch, einem Einschlauch-Ventilsystem oder einem Doppelschlauchsystem verwendet werden.

Beim Leckageschlauch wird über ein Ausatemsystem die kohlendioxidhaltige Ausatemluft kontinuierlich ausgespült.

Beim Einschlauch-Ventilsystem und beim Doppelschlauchsystem wird die Ausatmung des Patienten über ein Ventil 30 gesteuert.

Beim Doppelschlauchsystem ist das Ventil 30 im Gerät angeordnet. Die Ausatemluft wird über einen Teilschlauch zum exspiratorischen Eingangsstutzen 28' geleitet und von dort über das Ventil 30 in die Umgebung abgegeben. Das Ventil 30 öffnet dafür mit jeder Exspiration. Das Ventil 30 wird mit jeder Inspiration geschlossen.

Ein Druckmessschlauch 32 greift den Druck im Schlauchsystem ab. Der Steuerdruck für das Ventil 30 kommt aus der Vorrichtung 20 über einen Druckschlauch.

Beim Einschlauch-Ventilsystem ist das Ventil 30 im oder am Schlauch 31 angeordnet.

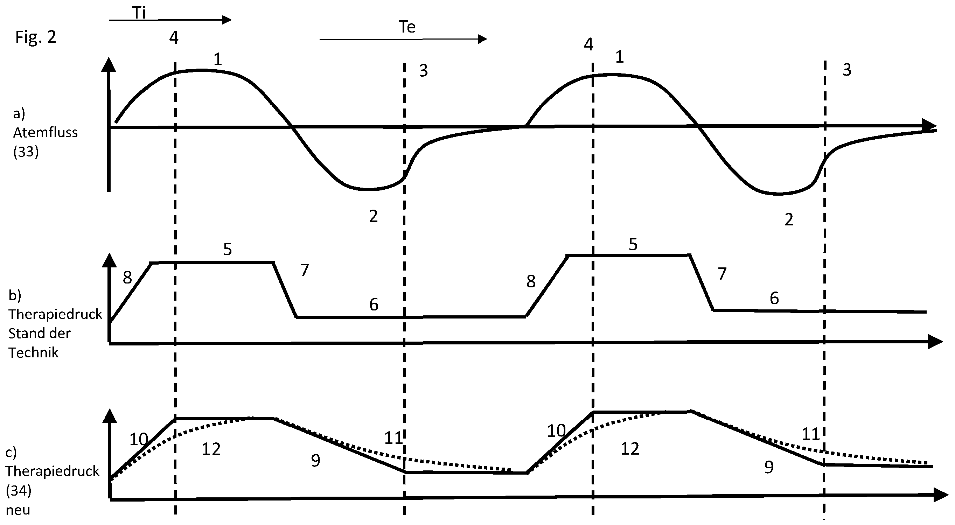

Fig. 2 a) zeigt einen Atemgasfluss 33, der sensorisch von der Vorrichtung 20 aufgezeichnet wurde. Man erkennt aus dem Flusssignal 33 die Inspiration 1 des Patienten und die Exspiration 2 des Patienten. Inspiration 1 und Exspiration 2 wechseln einander im Atemrhythmus des Patienten ab. Die Steuereinheit kann aus dem Flusssignal 33 den Wechsel von Inspiration 1 und Exspiration 2 erkennen und eine Druckvorgabe 34 entsprechend umsteuern. Die Steuereinheit kann aus dem Fluss den Wechsel von Inspiration und Exspiration erkennen und die Zeitdauern von Inspiration (Ti) und Exspiration (Te) bestimmen.

Fig. 2 b) und c) zeigen, dass die Vorrichtung im Wechsel von Inspiration und Exspiration den IPAP 5 bzw. den EPAP 6 vorgibt.

In Fig. 2 b) ist zu erkennen, dass bei Vorrichtungen nach dem Stand der Technik die Umsteuerung vom IPAP auf den EPAP zumeist über einen rampenförmigen Druckabfall 7 erfolgt. Dabei wird der EPAP kurz nach dem Atemphasenwechsel erreicht. Auch die Umsteuerung vom EPAP auf den IPAP erfolgt zumeist über einen rampenförmigen Druckanstieg 8. Der IPAP wird dann auch schnell erreicht. Die Rampe 7, 8 ist jeweils typischerweise mit fester Dauer oder Geschwindigkeit einstellbar oder vorgegeben.

In Fig. 2 c) steuert die Vorrichtung 20 den Druckabfall vom IPAP 5 auf den EPAP 6 so, dass der EPAP 6 nach der Hälfte der typischen Exspirationszeit 3 erreicht wird oder 90 % des Druckabfalls nach der Hälfte der typischen Exspirationszeit 3 erreicht ist. Die Vorrichtung 20 zur Beatmung weist dazu eine steuerbare Atemgasquelle 21 und eine programmierbare Steuereinheit 22 auf. Die programmierbare Steuereinheit 22 ist zum Durchführen der folgenden Schritte eingerichtet: Bestimmung des Atemgasflusses 33, wobei aus dem Atemgasfluss 33 bestimmt wird, ob eine Inspiration oder eine Exspiration bereitgestellt wird; Regelung des Drucks für eine Inspiration (IPAP) und eine Exspiration (EPAP), wobei die Steuereinheit 22 eine typische Exspirationszeit 3 über n Atemzüge ermittelt, wobei die Steuereinheit 22 den Druck vom IPAP 5 auf den EPAP 6 unter Berücksichtigung der typischen Exspirationszeit 3 derart absenkt, dass der Druckabfall auf den EPAP 6 zumindest zu 90 % bereits nach einem Anteil oder Bruchteil der typischen Exspirationszeit 3 erreicht wird.

In dem Beispiel der adaptiven Druckrampe 9 erreicht der Druck den EPAP nach 40 % bis 60 %, bevorzugt nach 50 % der typischen Exspirationszeit 3.

In dem Beispiel des nichtlinearen Abfalls 11 mit asymptotischer Näherung an den EPAP ist der Abfall auf den EPAP zu zumindest 80 %, bevorzugt zu 90 %, nach 40 % bis 60 %, bevorzugt nach 50 % der typischen Exspirationszeit 3 erreicht.

Die Umsetzung erfolgt beispielsweise so, dass die Steuereinheit die durchschnittliche Exspirationszeit 3 durch einen gewichteten Mittelwertfilter, der ca. die letzten 10 Atemzüge berücksichtigt, ermittelt. Der Druck wird vom IPAP auf den EPAP mit einer passenden Geschwindigkeit abgesenkt, sodass nach 50 % der mittleren Exspirationszeit der EPAP erreicht wird. So bleibt noch ausreichend Zeit, um die nicht kollabierten Areale der Lunge auf dem EPAP-Niveau zu entlüften. Die kollapsiblen Areale bleiben zumindest während eines Teils der Rampenzeit geöffnet und werden dadurch teilentlüftet.

Die Soll-Exspirationszeiten oder -Inspirationszeiten, auf die sich die Prozent-Zielwerte der EPAP- bzw. IPAP-Erreichung und damit die Rampensteilheiten beziehen, können aus dem spontanen Atemmuster des Patienten abgeleitet werden.

Die Soll-Exspirationszeiten oder -Inspirationszeiten, auf die sich die Prozent-Zielwerte der EPAP- bzw. IPAP-Erreichung und damit die Rampensteilheiten beziehen, können alternativ oder zusätzlich als ideale Inspirations- bzw. Exspirationszeiten vorgegeben werden.

Die Vorgabe kann dabei manuell erfolgen oder automatisch anhand mindestens eines der folgenden Parameter durchgeführt werden: Alter, Größe, Erkrankung, Therapieziel, Lungenvolumen, Lungencompliance, Lungenresistance, Atemanstrengung, Atemnot.

In Fig. 2 c) ist zudem zu erkennen, dass die Vorrichtung 20 den Druckanstieg auf den IPAP 5 so steuert, dass der IPAP nach 20 % bis 40 %, bevorzugt nach 30 % der typischen Inspirationszeit erreicht wird.

In dem Beispiel der adaptiven Druckrampe erreicht der Druck den IPAP nach 30 % der typischen Inspirationszeit.

Die Umsetzung erfolgt beispielsweise so, dass die Steuereinheit die typische Inspirationszeit 4 durch einen gewichteten Mittelwertfilter, der ca. die letzten 10 Atemzüge berücksichtigt, ermittelt. Der Druck wird vom EPAP auf den IPAP mit einer passenden Geschwindigkeit erhöht, sodass nach 30 % der typischen Inspirationszeit 4 der IPAP erreicht wird.

In dem Beispiel des nichtlinearen Abfalls 11 mit asymptotischer Näherung an den EPAP ist der Abfall auf den EPAP zu zumindest 80 %, bevorzugt zu 90 %, nach 20 % bis 40 %, bevorzugt nach 30 % der typischen Inspirationszeit erreicht.

### Fig. 2 c) zeigt

- 1: Inspiration des Patienten
- 2: Exspiration des Patienten
- 3: 50 % der typischen Exspirationszeit (Te)
- 4: 30 % der typischen Inspirationszeit (Ti)
- 5: IPAP-Druckniveau (inspiratorischer Druck)
- 6: EPAP-Druckniveau (exspiratorischer Druck)
- 7: Druckrampe IPAP zu EPAP, typischerweise mit fester Dauer oder Geschwindigkeit oder damit assoziierter Stufe einstellbar
- 8: Druckrampe EPAP zu IPAP, typischerweise mit fester Dauer oder Geschwindigkeit oder damit assoziierter Stufe einstellbar
- 9: Adaptive Druckrampe -erreicht den EPAP nach 50 % der typischen Exspirationszeit
- 10: Adaptive Druckrampe - erreicht den IPAP nach 30 % der typischen Inspirationszeit
- 11: Alternative Ausführungsform der Rampe; kein linearer Abfall, sondern ein nichtlinearer Abfall mit asymptotischer Näherung an den EPAP; charakteristische Kennzahl: z. B. 90 % des Abfalls erreicht nach 50 % der Exspirationszeit
- 12: Alternative Ausführungsform der Rampe; kein linearer Anstieg, sondern ein nichtlinearer Anstieg mit asymptotischer Näherung an den IPAP; charakteristische Kennzahl: z. B. 90 % des Anstiegs erreicht nach 30 % der Inspirationszeit

Die Steuerung der Exspirationsrampe erfolgt so flach wie möglich, damit die unteren Atemwege bei COPD-Patienten mit exspiratorischer Flusslimitation so lange wie möglich geschient bleiben und nicht kollabieren. Damit wird die Lunge besser entlüftet und es entsteht ein geringerer intrinsischer PEEP. Der Patient kann leichter atmen, da die Lunge weniger gedehnt bleibt, und den Trigger auslösen. Nach dem Abschalten des Beatmungsgeräts fällt das Atmen leichter.

Die Umsetzung erfolgt beispielsweise so, dass die durchschnittliche Exspirationszeit durch einen gewichteten Mittelwertfilter, der ca. die letzten 10 Atemzüge berücksichtigt, ermittelt wird. Der Druck wird vom IPAP auf den EPAP mit einer passenden Geschwindigkeit abgesenkt, sodass nach 50 % der mittleren Exspirationszeit der EPAP erreicht wird. So bleibt noch ausreichend Zeit, um die nicht kollabierten Areale der Lunge auf dem EPAP-Niveau zu entlüften. Die kollapsiblen Areale bleiben zumindest während eines Teils der Rampenzeit geöffnet und werden dadurch teilentlüftet.

## Patentansprüche

1. Vorrichtung (20) zur Beatmung, wobei die Vorrichtung eine steuerbare Atemgasquelle (21) und eine programmierbare Steuereinheit (22) aufweist, wobei die programmierbare Steuereinheit (22) zum Durchführen der folgenden Schritte eingerichtet ist:
Bestimmen eines Atemgasflusses (33), wobei aus dem Atemgasfluss (33) bestimmt wird, ob eine Inspiration oder eine Exspiration vorliegt;
Regeln eines Drucks für die Inspiration (IPAP) und die Exspiration (EPAP);
**dadurch gekennzeichnet, dass** die Steuereinheit (22) eine typische Exspirationszeit (3) berücksichtigt, wobei die Steuereinheit (22) die typische Exspirationszeit über n Atemzüge ermittelt und die so ermittelte Exspirationszeit berücksichtigt, wobei die Steuereinheit (22) den Druck vom IPAP (5) auf den EPAP (6) unter Berücksichtigung der typischen Exspirationszeit (3) derart absenkt, dass der Druckabfall auf den EPAP (6) zumindest zu 85 % bereits nach einem Anteil der typischen Exspirationszeit (3) erreicht wird, der im Bereich von 40 % bis 60 % der typischen Exspirationszeit (3) liegt, wobei der EPAP (6) nach Beendigung des Druckabfalls bis zum Ende der typischen Exspirationszeit (3) vorgegeben wird.

2. Vorrichtung nach Anspruch 1, wobei der Druck vom IPAP auf den EPAP in Form einer adaptiven Druckrampe (9) abgesenkt wird, wobei der Druck den EPAP nach 40 % bis 60 %, bevorzugt nach 50 % der typischen Exspirationszeit (3) erreicht.

3. Vorrichtung nach mindestens einem der vorhergehenden Ansprüche, wobei der EPAP nach einem Anteil der typischen Exspirationszeit (3) erreicht wird, der bei 50 % liegt.

4. Vorrichtung nach mindestens einem der vorhergehenden Ansprüche, wobei nach Erkennung einer Ausatemphase der Druck vom IPAP auf den EPAP so verringert wird, dass der Druck als dynamisch geregelter Gegendruck gegen den Atemgasfluss der Exspiration angelegt wird, wodurch die unteren Atemwege bei COPD-Patienten mit exspiratorischer Flusslimitation für etwa die Hälfte der typischen Exspirationszeit (3) geschient bleiben und nicht kollabieren.

5. Vorrichtung nach mindestens einem der vorhergehenden Ansprüche, wobei der Druck vom IPAP auf den EPAP als ein nichtlinearer Abfall mit asymptotischer Näherung an den EPAP verringert wird.

6. Vorrichtung nach mindestens einem der vorhergehenden Ansprüche, wobei der Druck vom IPAP auf den EPAP so verringert wird, dass 90 % des Druckabfalls nach etwa der Hälfte der Exspirationszeit erreicht ist.

7. Vorrichtung nach mindestens einem der vorhergehenden Ansprüche, wobei der Druck nur zu Beginn der Exspiration geregelt wird und nach Erreichen des EPAP auf diesem Niveau belassen wird.

8. Vorrichtung nach mindestens einem der vorhergehenden Ansprüche, wobei die Steuereinheit dazu eingerichtet und ausgebildet ist, eine Erhöhung des exspiratorischen Widerstands zu erkennen und den Druckabfall vom IPAP (5) auf den EPAP (6) unter Berücksichtigung des exspiratorischen Widerstands, beispielsweise indem die Rampe des Druckabfalls flacher eingestellt wird, durchzuführen.

9. Vorrichtung nach mindestens einem der vorhergehenden Ansprüche, wobei die Steuereinheit dazu eingerichtet und ausgebildet ist, einen erhöhten Restfluss zum Ende der Exspiration zu erkennen und den Druckabfall vom IPAP (5) auf den EPAP (6) unter Berücksichtigung des erhöhten Restflusses zum Ende der Exspiration, beispielsweise indem die Rampe des Druckabfalls steiler eingestellt wird, durchzuführen.

10. Vorrichtung nach mindestens einem der vorhergehenden Ansprüche, wobei die Steuereinheit dazu eingerichtet und ausgebildet ist, eine Variation der Steilheit des Druckabfalls, beispielsweise von Atemzug zu Atemzug, durchzuführen und zu ermitteln, bei welcher Steilheit des Druckabfalls die beste Entlüftung der Lunge erfolgt.

11. Vorrichtung nach mindestens einem der vorhergehenden Ansprüche, wobei die Steuereinheit dazu eingerichtet und ausgebildet ist, eine Änderung der Steilheit des Druckabfalls, beispielsweise nach wie viel Prozent der Exspirationszeit der EPAP erreicht sein soll, entsprechend einer Vorgabe, die über eine Datenschnittstelle eingegeben wird, durchzuführen.

12. Vorrichtung nach mindestens einem der vorhergehenden Ansprüche, wobei die Steuereinheit dazu eingerichtet und ausgebildet ist, eine Änderung der Steilheit des Druckabfalls basierend auf sensorischen Signalen, beispielsweise von Effort-Gurten, Zwerchfell-EMG, Ösophagusdruck-Sonden, Bodyplethysmografie oder Elektroimpedanztomografie, zu steuern.

13. Vorrichtung nach mindestens einem der vorhergehenden Ansprüche, wobei die Steuereinheit dazu eingerichtet und ausgebildet ist, eine Änderung der Steilheit des Druckabfalls durchzuführen, wenn die Druckunterstützung angepasst wird, also die Differenz zwischen IPAP und EPAP geändert wird.

14. Vorrichtung nach mindestens einem der vorhergehenden Ansprüche, wobei die Steuereinheit dazu eingerichtet und ausgebildet ist, eine Änderung der Steilheit des Druckabfalls durchzuführen, wenn der EPAP angepasst wird.

15. Vorrichtung nach mindestens einem der vorhergehenden Ansprüche, wobei die Steuereinheit (22) eine typische Inspirationszeit (4) über n Atemzüge ermittelt und den Druck vom EPAP (6) auf den IPAP (5) unter Berücksichtigung der typischen Inspirationszeit (4) derart anhebt, dass der IPAP (5) nach einem Anteil der typischen Inspirationszeit (4) erreicht wird, der im Bereich von 20 % bis 40 % oder im Bereich von 30 % liegt.

16. Vorrichtung nach Anspruch 15, wobei die Steuereinheit (22) den Druck vom EPAP (6) auf den IPAP (5) derart anhebt, dass etwa 90 % des Anstiegs nach 30 % der typischen Inspirationszeit erreicht ist.

17. Vorrichtung nach Anspruch 15 oder 16, wobei die Steuereinheit (22) die typische Inspirationszeit (4) über zumindest 3 oder bevorzugt 10 Atemzüge ermittelt, wobei ein Atemzug eine Inspiration und eine Exspiration umfasst.

18. Vorrichtung nach mindestens einem der vorhergehenden Ansprüche, wobei die Steuereinheit (22) die typische Exspirationszeit (3) über zumindest 3 oder bevorzugt 10 Atemzüge ermittelt, wobei ein Atemzug eine Inspiration und eine Exspiration umfasst.

19. Vorrichtung nach mindestens einem der vorhergehenden Ansprüche, wobei die Steuereinheit (22) den Druck vom EPAP (6) auf den IPAP (5) derart anhebt, dass ein nichtlinearer Anstieg mit asymptotischer Näherung an den IPAP resultiert.

20. Vorrichtung nach mindestens einem der vorhergehenden Ansprüche, wobei die Druckveränderung vom EPAP zum IPAP oder vom IPAP zum EPAP mit fester Dauer oder Geschwindigkeit oder einer damit assoziierten Stufe vorgebbar ist.

21. Vorrichtung nach mindestens einem der vorhergehenden Ansprüche, wobei das Atemvolumen der Inspiration (AI) bestimmt wird und die Zeitdauer der Exspiration angepasst wird, wenn das Atemvolumen der Exspiration (AE) den Wert des Atemvolumens der Inspiration (AI) erreicht.

## Claims

1. A device (20) for ventilation, wherein the device has a controllable breathing gas source (21) and a programmable control unit (22), wherein the programmable control unit (22) is configured to carry out the following steps:
determining a breathing gas flow (33), wherein it is determined from the breathing gas flow (33) whether inspiration or expiration is occurring;
regulating a pressure for the inspiration (IPAP) and the expiration (EPAP);
**characterized in that** the control unit (22) takes into account a typical expiration time (3), wherein the control unit (22) ascertains the typical expiration time over n breaths and the expiration time ascertained in this way is taken into account, wherein the control unit (22) lowers the pressure from the IPAP (5) to the EPAP (6) taking into account the typical expiration time (3) such that the pressure drop to the EPAP (6) is already achieved at least by 85% after a proportion of the typical expiration time (3) that lies in the range of from 40% to 60% of the typical expiration time (3), wherein the EPAP (6) is specified after completion of the pressure drop until the end of the typical expiration time (3).

2. The device according to claim 1, wherein the pressure is lowered from the IPAP to the EPAP in the form of an adaptive pressure ramp (9), wherein the pressure reaches the EPAP after 40% to 60%, preferably after 50% of the typical expiration time (3).

3. The device according to at least one of the preceding claims, wherein the EPAP is reached after a proportion of the typical expiration time (3) of 50%.

4. The device according to at least one of the preceding claims, wherein, after an exhalation phase has been recognized, the pressure is reduced from the IPAP to the EPAP in such a way that the pressure is applied as a dynamically regulated back pressure against the breathing gas flow during expiration, as a result of which the lower respiratory tract in COPD patients with expiratory flow limitation remains splinted and does not collapse for approximately half of the typical expiration time (3).

5. The device according to at least one of the preceding claims, wherein the pressure is reduced from the IPAP to the EPAP as a non-linear drop with asymptotic approximation to the EPAP.

6. The device according to at least one of the preceding claims, wherein the pressure is reduced from the IPAP to the EPAP in such a way that 90% of the pressure drop is achieved after approximately half of the expiration time.

7. The device according to at least one of the preceding claims, wherein the pressure is only regulated at the start of expiration and is kept at the level of the EPAP after same has been achieved.

8. The device according to at least one of the preceding claims, wherein the control unit is configured and designed to recognize an increase in the expiratory resistance and carries out the pressure drop from the IPAP (5) to the EPAP (6) taking into account the expiratory resistance, for example by setting the pressure drop ramp to be flatter.

9. The device according to at least one of the preceding claims, wherein the control unit is configured and designed to recognize an increased residual flow at the end of expiration and to carry out the pressure drop from the IPAP (5) to the EPAP (6) taking into account the increased residual flow at the end of expiration, for example by setting the pressure drop ramp to be steeper.

10. The device according to at least one of the preceding claims, wherein the control unit is configured and designed to vary the steepness of the pressure drop, for example on a breath-by-breath basis, and to ascertain at which steepness of the pressure drop the best deflation of the lungs takes place.

11. The device according to at least one of the preceding claims, wherein the control unit is configured and designed to change the steepness of the pressure drop, for example after what percentage of the expiration time the EPAP should be achieved, in accordance with a specification that is entered via a data interface.

12. The device according to at least one of the preceding claims, wherein the control unit is configured and designed to control a change in the steepness of the pressure drop based on sensor signals, for example from effort belts, diaphragmatic EMG, esophageal pressure probes, body plethysmography or electrical impedance tomography.

13. The device according to at least one of the preceding claims, wherein the control unit is configured and designed to change the steepness of the pressure drop if the pressure support is adapted, i.e. the difference between the IPAP and EPAP is changed.

14. The device according to at least one of the preceding claims, wherein the control unit is configured and designed to change the steepness of the pressure drop if the EPAP is adapted.

15. The device according to at least one of the preceding claims, wherein the control unit (22) ascertains a typical inspiration time (4) over n breaths and raises the pressure from the EPAP (6) to the IPAP (5) taking into account the typical inspiration time (4) such that the IPAP (5) is achieved after a proportion of the typical inspiration time (4) that lies in the range of from 20% to 40% or in the region of 30%.

16. The device according to claim 15, wherein the control unit (22) raises the pressure from the EPAP (6) to the IPAP (5) in such a way that approximately 90% of the increase is achieved after 30% of the typical inspiration time.

17. The device according to claim 15 or 16, wherein the control unit (22) ascertains the typical inspiration time (4) over at least 3 or preferably 10 breaths, wherein one breath comprises one inspiration and one expiration.

18. The device according to at least one of the preceding claims, wherein the control unit (22) ascertains the typical expiration time (3) over at least 3 or preferably 10 breaths, wherein one breath comprises one inspiration and one expiration.

19. The device according to at least one of the preceding claims, wherein the control unit (22) raises the pressure from the EPAP (6) to the IPAP (5) in such a way that a non-linear increase with asymptotic approximation to the IPAP results.

20. The device according to at least one of the preceding claims, wherein the pressure change from the EPAP to the IPAP or from the IPAP to the EPAP can be specified with a fixed duration or speed or an associated level.

21. The device according to at least one of the preceding claims, wherein the respiratory volume of the inspiration (AI) is determined and the duration of the expiration is adapted if the respiratory volume of the expiration (AE) reaches the value of the respiratory volume of the inspiration (AI).

## Revendications

1. Dispositif (20) de ventilation, dans lequel le dispositif présente une source de gaz respiratoire commandable (21) et une unité de commande programmable (22), dans lequel l'unité de commande programmable (22) est configurée pour la mise en œuvre des étapes suivantes:
détermination d'un flux de gaz respiratoire (33), dans lequel le flux de gaz respiratoire (33) permet de déterminer la présence d'une inspiration ou d'une expiration;
réglage d'une pression pour l'inspiration (IPAP) et l'expiration (EPAP);
**caractérisé en ce que** l'unité de commande (22) tient compte d'un temps d'expiration typique (3), dans lequel l'unité de commande (22) détermine le temps d'expiration typique sur n respirations et tient compte du temps d'expiration ainsi déterminé, dans lequel l'unité de commande (22) abaisse la pression de l'IPAP (5) à l'EPAP (6) en tenant compte du temps d'expiration typique (3) de telle façon que la baisse de pression à l'EPAP (6) est atteinte à au moins 85 % déjà après un pourcentage du temps d'expiration typique (3) compris dans la plage de 40 % à 60 % du temps d'expiration typique (3), dans lequel l'EPAP (6) est prédéfinie après l'achèvement de la baisse de pression jusqu'à la fin du temps d'expiration typique (3).

2. Dispositif selon la revendication 1, dans lequel la pression est abaissée de l'IPAP à l'EPAP sous la forme d'une rampe de pression adaptative (9), dans lequel la pression atteint l'EPAP après 40 % à 60 %, de préférence après 50 % du temps d'expiration typique (3).

3. Dispositif selon l'une au moins des revendications précédentes, dans lequel l'EPAP est atteinte après un pourcentage du temps d'expiration typique (3) s'élevant à 50 %.

4. Dispositif selon l'une au moins des revendications précédentes, dans lequel, après la reconnaissance d'une phase expiratoire, la pression est réduite de l'IPAP à l'EPAP de telle façon que la pression est appliquée en tant que contre-pression réglée dynamiquement contre le flux de gaz respiratoire de l'expiration, moyennant quoi les voies respiratoires inférieures sont maintenues ouvertes et ne s'affaissent pas sur environ la moitié du temps d'expiration typique (3) chez des patient atteints de BPCO avec une limitation de flux expiratoire.

5. Dispositif selon l'une au moins des revendications précédentes, dans lequel la pression est réduite de l'IPAP à l'EPAP en tant que baisse non linéaire avec une approximation asymptomatique de l'EPAP.

6. Dispositif selon l'une au moins des revendications précédentes, dans lequel la pression est réduite de telle façon de l'IPAP à l'EPAP que 90 % de la baisse de pression sont atteints après environ la moitié du temps d'expiration.

7. Dispositif selon l'une au moins des revendications précédentes, dans lequel la pression est réglée seulement au début de l'expiration et maintenue à ce niveau après atteinte de l'EPAP.

8. Dispositif selon l'une au moins des revendications précédentes, dans lequel l'unité de commande est configurée et conçue pour reconnaître une augmentation de la résistance expiratoire et pour effectuer la baisse de pression de l'IPAP (5) à l'EPAP (6) en tenant compte de la résistance expiratoire, par exemple en réglant la rampe de la baisse de pression de façon plus plane.

9. Dispositif selon l'une au moins des revendications précédentes, dans lequel l'unité de commande est configurée et conçue pour reconnaître un flux résiduel élevé à la fin de l'expiration et pour effectuer la baisse de pression de l'IPAP (5) à l'EPAP (6) en tenant compte du flux résiduel élevé à la fin de l'expiration, par exemple en réglant la rampe de la baisse de pression de façon plus raide.

10. Dispositif selon l'une au moins des revendications précédentes, dans lequel l'unité de commande est configurée et conçue pour effectuer une variation de la pente de la baisse de pression, par exemple de respiration en respiration, et pour déterminer à quelle pente de la baisse de pression a lieu la meilleure aération des poumons.

11. Dispositif selon l'une au moins des revendications précédentes, dans lequel l'unité de commande est configurée et conçue pour effectuer une modification de la pente de la baisse de pression, par exemple le pourcentage du temps d'expiration après lequel l'EPAP doit être atteinte, en fonction d'une consigne entrée par le biais d'une interface de données.

12. Dispositif selon l'une au moins des revendications précédentes, dans lequel l'unité de commande est configurée et conçue pour commander une modification de la pente de la baisse de pression sur la base de signaux sensoriels, par exemple de ceintures d'effort, d'une électromyographie du diaphragme, de sondes de pression œsophagienne, d'une pléthysmographie pneumologique ou d'une tomographie par impédance électrique.

13. Dispositif selon l'une au moins des revendications précédentes, dans lequel l'unité de commande est configurée et conçue pour effectuer une modification de la pente de la baisse de pression lorsque l'assistance de pression est adaptée, donc lorsque la différence entre l'IPAP et l'EPAP est modifiée.

14. Dispositif selon l'une au moins des revendications précédentes, dans lequel l'unité de commande est configurée et conçue pour effectuer une modification de la pente de la baisse de pression lorsque l'EPAP est adaptée.

15. Dispositif selon l'une au moins des revendications précédentes, dans lequel l'unité de commande (22) détermine un temps d'inspiration typique (4) sur n respirations et augmente la pression de l'EPAP (6) à l'IPAP (5) en tenant compte du temps d'inspiration typique (4) de telle façon que l'IPAP (5) est atteinte après un pourcentage du temps d'inspiration typique (4) compris dans la plage de 20 % à 40 % ou dans la plage de 30 %.

16. Dispositif selon la revendication 15, dans lequel l'unité de commande (22) augmente la pression de l'EPAP (6) à l'IPAP (5) de telle façon qu'environ 90 % de la hausse sont atteints après 30 % du temps d'inspiration typique.

17. Dispositif selon la revendication 15 ou 16, dans lequel l'unité de commande (22) détermine le temps d'inspiration typique (4) sur au moins trois ou de préférence 10 respirations, dans lequel une respiration comporte une inspiration et une expiration.

18. Dispositif selon l'une au moins des revendications précédentes, dans lequel l'unité de commande (22) détermine le temps d'expiration typique (3) sur au moins 3 ou de préférence 10 respirations, dans lequel une respiration comporte une inspiration et une expiration.

19. Dispositif selon l'une au moins des revendications précédentes, dans lequel l'unité de commande (22) augmente la pression de l'EPAP (6) à l'IPAP (5) de manière à obtenir une hausse non linéaire avec une approximation asymptomatique de l'IPAP.

20. Dispositif selon l'une au moins des revendications précédentes, dans lequel la modification de pression de l'EPAP à l'IPAP ou de l'IPAP à l'EPAP peut être prédéfinie avec une durée ou une vitesse fixe ou avec un étage associé à celle-ci.

21. Dispositif selon l'une au moins des revendications précédentes, dans lequel le volume respiratoire de l'inspiration (AI) est déterminé et la durée de l'expiration est adaptée lorsque le volume respiratoire de l'expiration (AE) atteint la valeur du volume respiratoire de l'inspiration (AI).
